# EUROPÄISCHE PATENTSCHRIFT

(11) **EP 1 868 500 B1**
(45) Veröffentlichungstag und Bekanntmachung des Hinweises auf die Patenterteilung: **21.10.2009**
(21) Anmeldenummer: 06724086.1
(22) Anmeldetag: 06.04.2006
(51) Int. Cl.: A61B 5/151

(54) **VERFAHREN UND VORRICHTUNG ZUR ENTNAHME VON KÖRPERFLÜSSIGKEIT**
METHOD AND DEVICE FOR THE EXTRACTION OF A BODY FLUID
PROCEDE ET DISPOSITIF POUR EXTRAIRE UN LIQUIDE CORPOREL

(30) Priorität: 07.04.2005 EP 05007580
(43) Veröffentlichungstag der Anmeldung: 26.12.2007
(73) Patentinhaber: F. Hoffmann-La Roche AG, 4070 Basel (CH); Roche Diagnostics GmbH, 68305 Mannheim (DE)
(72) Erfinder: SAROFIM, Emad, 6332 Hagendorn (CH); CALASSO, Irio, Giuseppe, CH-6415 Arth (CH); KORNER, Stephan, CH-6330 Cham (CH); ABT, Reto, 5624 Bünzen (CH); HEIN, Heinz-Michael, 6044 Udligenswil (CH); JAEGGI, Rainer, 8800 Thalwil (CH); GRISS, Patrick, 8112 Otelfingen (CH)
(74) Vertreter: Pfiz, Thomas
(86) Internationale Anmeldenummer: PCT/EP2006/003141
(87) Internationale Veröffentlichungsnummer: WO 2006/105968

(56) Entgegenhaltungen:
- EP-A- 1 238 632
- WO-A-03/009759
- WO-A-20/04080306
- US-A- 5 857 983
- US-A1- 2002 042 594
- US-A1- 2002 168 290

## Beschreibung

Die Erfindung betrifft eine Vorrichtung zur Entnahme von Körperflüssigkeit, insbesondere Blut, gemäß dem Oberbegriff des Patentanspruchs 1.

Solche Entnahmevomchtungen für kleine Körperflüssigkeitsmengen dienen vor allem Diabetikern zur täglich mehrfach durchgeführten Blutzucker-Selbstkontrolle. Neuere Konzepte sehen eine messende MIkronadel als Einwegartikel (Disposable) in einem Handgerät vor, um einen Hauteinstich zu erzeugen, daraus eine kleine Menge Blut unter Ausnutzung von Kapillarkräf ten zu entnehmen und diese Blutprobe zu analysieren. Mit einem solchen integrierten System sollen in einem weitgehend automatischen Messablauf auch von Laien die erforderlichen Schritte einfach und schnell vorgenommen werden können. Um dies zu erreichen, ist es wichtig, dass Stech- und Sammelvorgang geräteseitig auf das Disposable abgestimmt sind, damit effizient, schnell und schmerzfrei Blut oder gegebenenfalls Gewebeflüssigkeit gesammelt werden kann.

In diesem Zusammenhang wurde bereits vorgeschlagen, zur Steuerung der vorwärts gerichteten Einstechbewegung einen Hautkontakt zu erfassen, um so eine definierte Einstechtiefe erreichen zu können. Eine solche Bewegungssteuerung muss aber mit großern apparativem Aufwand an die gewünschte hohe Einstechgeschwindigkeit angepasst werden. Vor allem ist aber nachteilig, dass damit keine Erkenntnis über einen tatsächlichen Erfolg der Blutaufnahme gewonnen werden kann.

Zur Optimierung der Blutgewinnung beschreibt die WO 03/009759 A1 der Anmelderin ein Kombinationsverfahren, bei dem zunächst eingestochen wird, die Stecheinheit mit Kapillarstruktur sodann um einen Teil der Einstichstrecke zurückgezogen und dort für eine Sammelperiode von einigen Sekunden verharren gelassen wird. Dadurch soll ein Teil des Stichkanals freigegeben werden, so dass sich Körperflüssigkeit darin sammeln kann und von dort in die Kapillarstruktur eindringt.

Aus der WO2004/080306 ist ein Stechsystem bekannt, welches dazu ausgebildet ist, eine charakteristische Größe (z.B. Impedanz) im Zuge der Stechbewegung und gegebenenfalls auch noch beim Zurückziehen zu erfassen. Dies soll allerdings nur einer Hauterkennung als solcher dienen, um gegebenenfalls die Eindringtiefe oder Einddngstabilität bzw. die Verweildauer dem Benutzer anzuzeigen. Eine Erfolgskontrolle für einen tatsächlichen Flüssigkeitskontakt über die Hautdetektion hinaus ist hingegen an keiner Stelle angesprochen.

Ausgehend hiervon liegt der Erfindung die Aufgabe zugrunde, die im Stand der Technik aufgetretenen Nachteile zu vermeiden und bei einfacher Benutzung eine insbesondere hinsichtlich Zuverlässigkeit, Effizienz, Schmerzminimierung und Hygiene optimierte Gewinnung der Körperflüssigkeit zu ermöglichen.

Zur Lösung dieser Aufgabe wird die in den unabhängigen Patentansprüchen jeweils angegebene Merkmalskombination vorgeschlagen.

Ein besonderer Efindungsaspekt Iiegt darin, den Sammelvorgang nicht sta-tisch durchzuführen, sondern in einer schmerzarmen Zone einen verbesserten Blutzufluss zu gewährleisten. Dementsprechend wird vorgeschlagen, dass das Stechelement nach der Vorwärtsbewegung in einer Rückziehphase um einen ersten Weg und in einer späteren Sammelphase um einen zweiten Weg zurückgezogen wird, wobei die Rückziehphase kürzer als die Sammelphase und der erste Weg größer als der zweite Weg und der zweite Weg größer als Null ist. Eine entsprechende Vorrichtung sieht vor, dass der Stechantrieb zur Rückbewegung des Stechelements in einer Rückziehphase um einen ersten Weg und in einer späteren Sammelphase um einen zweiten Weg ausgebildet ist, wobei die Rückziehphase kürzer als die Sammelphase und der erste Weg größer als der zweite Weg und der zweite Weg größer als Null ist. Damit kann rasch und schmerzarrn ein Stichkanal in einer Blutzone geschaffen werden, während der Sammelvorgang mit der erforderlichen Dauer für den Selbsttransport der Flüssigkeit in der Kapillarstruktur in einer unempfindlicheren äußeren Hautzone erfolgt. Überraschend hat sich gezeigt, dass durch das langsame Zurückziehen die Sammeleffizienz erheblich besser ist als bei statischen Sammelpositlonen. Eine mögliche Erklärung kann darin liegen, dass die Haut nach der schnellen Stechbewegung durch das Stechorgan eingedrückt ist und diese Andruckkraft zunächst das Fließen des Blutes verhindert. Beim langsamen Zurückziehen während der Sammelphase wird jedoch die Haut wieder entspannt, so dass Blut besser Nachfließen kann. Denkbar ist es auch, dass durch das Zurückziehen des Stechelements eine Art Saugeffekt erreicht wird, so dass zusätzliches Blut zur Verfügung steht. Dabei ist das Sammeln im eingestochenen Zustand hygienisch günstig und für die dosierte Entnahme kleiner Flüssigkeitsmengen effizient.

Vorteilhafte Ausgestaltungen und Weiterbildungen der Erfindung ergeben sich aus den abhängigen Ansprüchen.

Durch die rasche Rückbewegung in eine Rückziehposition von geringerer Stechtiefe wird der Schmerzbereich des Körperteils bzw. der Haut schnell verlassen. In dieser äußeren nicht-blutgebenden Zone wird dann nach einer Wartezeit kontrolliert, ob Blut über den Stichkanal nachfliesst. Bei Flüssigkeitskontakt kann von einer hohen Sammelerfolgsrate ausgegangen werden, ohne überflüssiges Totvolumen. Außerdem kann noch in der Haut besonders hygienisch Flüssigkeit aufgenommen werden, ohne dass austretendes Blut zu sehen wäre. Zur Erfassung des Flüssigkeitskontakts eignet sich vor allem eine Impedanzmessung unter Einbeziehung des Stechorgans als Elektrode. Nach Maßgabe eines erfassten Körperflüssigkeitskontakts lässt sich der Erhalt von KörperFlüssigkeit aus dem Körperteil bzw. aus der Stichwunde kontrollieren. Die Wahrscheinlichkeit für eine erfolgreiche Aufnahme der Flüssigkeit wird durch eine solche Kontrolle erheblich erhöht. Hierdurch kann auch die optimale Sammelzeit abgewartet werden. Gegebenenfalls wird bei ausbleibendem Kontakt ein Fehlersignal generiert und der Entnahmevorgang abgebrochen.

Besonders bevorzugt wird die Körperflüssigkeit nach dem schnellen Zurückziehen des Stechelements während einer Sammelphase in der äußeren schmerzarmen Hautschicht aufgenommen. Dabei wird das Stechelement zumindest während eines Teils der Sammelphase gleichförmig oder mit veränderlicher Geschwindigkeit zurückgezogen, wodurch die Sammeleffizienz entscheidend verbessert wird. Die Sammelzeit sollte ausreichend bemessen sein, um unter der kapillaraktiven Wirkung eine hinreichende Flüssigkeitsaufnahme sicherzustellen.

Insbesondere sind alle offenbarten Ausführungsformen hinsichtlich der Körperflüssigkeitserfassung und den Stechprofilen miteinander kombinierbar.

Im Folgenden wird die Erfindung anhand der in der Zeichnung schematisch dargestellten Ausführungsbeispiele näher erläutert. Es zeigen
- Fig. 1: ein Blockschaltbild einer Vorrichtung zur Entnahme und ggf. Analyse von Blut aus einem Körperteil;
- Fig. 2 bis 5: ein Stechelement der Vorrichtung nach Fig. 1 in verschiedenen Stechpositionen in vereinfachter Schrittdarstellung;
- Fig. 6 bis 9: verschiedene Stechprofile beim Einsatz des Stechelements;
- Fig. 10: die Anatomie der menschlichen Haut in einer schaubildlichen Darstellung;
- Fig.11: den Signalverlauf in verschiedenen Konstellationen eines Körperflüssigkeitskontakts.

Die in der Zeichnung dargestellte Vorrichtung dient der Selbstentnahme einer Blutprobe durch einen Benutzer für Analysezwecke, insbesondere zur Blutzuckerkontrolle. Die Vorrichtung umfasst ein Stechelement 10 als Einmalartikel zur Blutaufnahme und ein Handgerät 12 zur automatischen Handhabung des eingesetzten Stechelements 10.

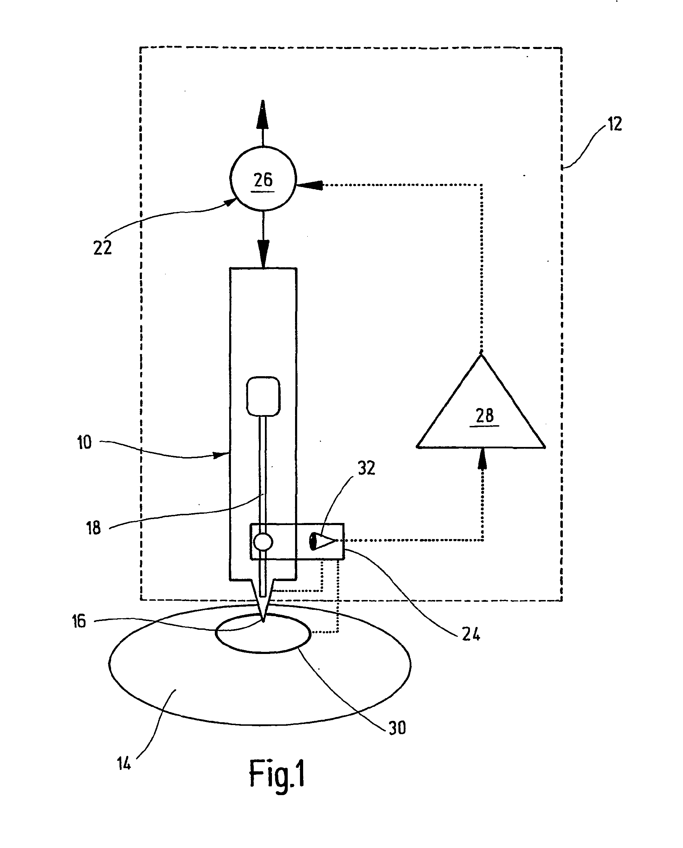

Das in Fig. 1 gezeigte Stechelement 10 ist als so genannter "Microsampler" zur Gewinnung einer kleinen Blutmenge aus einem Körperteil 14, insbesondere einer Fingerkuppe vorgesehen. Es besteht als Flachformteil aus dünnem Edelstahlblech und weist eine distal angeformte Spitze 16 als Stechorgan auf, die über einen halboffenen rillenförmigen Kapillarkanal 18 mit einer Sammelstelle 20 verbunden ist, welche als Reaktionsbereich für einen Nachweis eines Analyten, z. B. Glucose ausgebildet sein kann. Der Blutglucosenachweis speziell mittels berührungsloser optischer Methoden ist im Stand der Technik an sich bekannt und wird daher hier nicht näher erläutert. Optional kann das gesammelte Blut aber auch in eine Analyseeinheit transferiert werden, um dort den Analyten zu bestimmen.

Das Gerät bzw. Instrument 12 weist einen Stechantrieb 22 für eine kontrollierte Vorwärts- und Rückbewegung des Stechelements 10 und Detektionsmittel 24 zur Erfassung eines Blutkontakts des Stechelements 10 während einer Sammelphase auf. Der Stechantrieb 22 umfasst eine mechanisch und/oder elektrisch arbeitende Antriebseinheit .26, die mit dem Stechelement 10 gekoppelt ist. Speziell kann ein zweistufiger Hybridantrieb vorgesehen sein, bei dem eine schnelle Bewegung von einer Mechanik, beispielsweise einer Antriebsfeder oder Antriebskulisse übernommen wird, während ein langsamere geregelte Bewegung über einen Elektromotor erfolgt. Alternativ kann auch ein elektromagnetischer Antrieb nach Art einer Lautsprecherspule den Stechhub bewirken. Der Stechantrieb 22 umfasst ferner eine Steuereinheit 28 für die Ablaufsteuerung oder -regelung der Blutentnahme und insbesondere der Stechbewegung. Zweckmäßig wird hierfür ein Mikrocontroller eingesetzt, der gegebenenfalls zusätzlich benutzerspezifisch parametrisiert werden kann.

Die Detektionsmittel 24 können den Blutkontakt während der Sammelphase durch eine Impedanzmessung über das Stechorgan 16 und einen von dem Stechorgan axial durchsetzten und in Anlage mit dem Körperteil 14 befindlichen Andruckring 30 als Gegenelektrode erfassen. Das aus elektrisch leitfähigem Material bestehende Stechorgan kann hierbei mit einem elektrischen Wechselspannungssignal, z.B. 4 Vpp und 10 kHz gefahrlos angesteuert werden, wobei durch einen fluidischen Kontakt die Impedanz in dem Pfad zwischen Stechorgan und Gegenelektrode signifikant abnimmt. Eine Kontrolleinrichtung 32 ermöglicht eine Erfolgskontrolle der Blutaufnahme in Abhängigkeit von dem erfassten Flüssigkeitskontakt, wie es nachstehend näher erläutert wird.

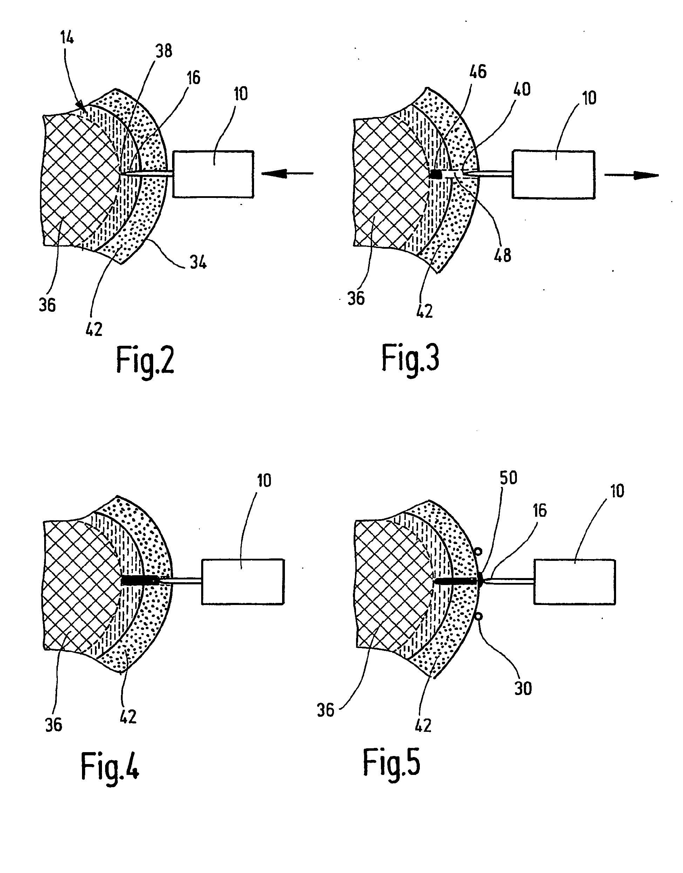

Die Fig. 2 bis 5 veranschaulichen den Ablauf der Blutgewinnung mittels des Stechelements 10. Zunächst wird in einer distal gerichteten Vorwärtsbewegung das Stechorgan 16 durch die Hautoberfläche 34 hindurch in das Körperteil 14 auf eine vorgegebene Tiefe eingestochen, so dass eine blutgebende Zone 36 in der Dermis erreicht wird. In diesem Bereich enden die Blutkapillaren, die durch das Stechorgan geöffnet werden können. Zugleich ist aber in diesem tieferen Bereich 36 aufgrund der dort vorhandenen Nervenzellen auch das Schmerzempfinden hoch. Aus diesem Grund ist es günstig, wenn das Stechelement unmittelbar nach der Vorwärtsbewegung in einer Rückziehbewegung aus der tiefsten Einstechposition 38 in eine Rückziehposition 40 von geringerer Stechtiefe zurückgezogen wird (Fig. 3). Bevorzugt liegt diese Rückziehposition im Bereich der Epidermis 42, speziell im Stratum Corneum 44. Nach dem schnellen Zurückziehen des Stechelements 10 verstreicht eine gewisse Zeit, bis das Blut 46 über den erzeugten Stichkanal 48 in die Epidermis nachfliest.

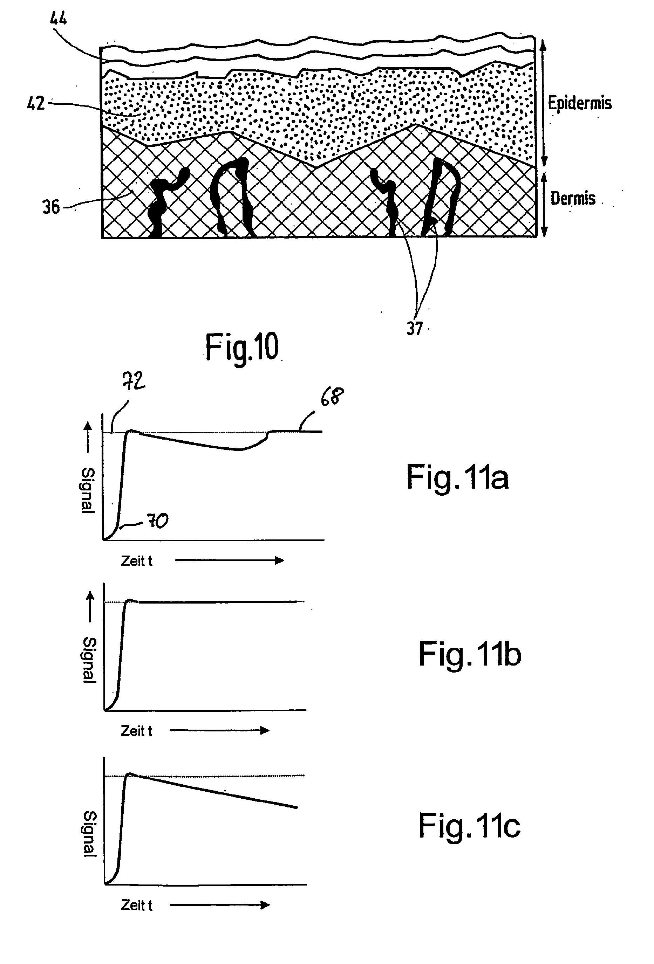

Die Dimensionen des Schichtaufbaus der menschlichen Haut sind in Fig. 10 realistischer veranschaulicht. Die Epidermis mit dem Stratum Corneum als oberster Hautschicht umfasst etwa 0,5 bis 1 mm Tiefe, während die Blutkapillaren 37 enthaltende Dermis in einen Tiefenbereich von einigen Millimetern erreicht.

Gemäß Fig. 4 wird in der zurückgezogenen Sammelposition 40 der Blutkontakt nach einer Wartezeit nach der Vorwärtsbewegung durch die Detektionsmittel 24 im Sinne einer Erfolgskontrolle erfasst. Auf diese Weise kann die Sammelzeit optimiert werden, da bei einem Flüssigkeitskontakt mit Sicherheit Blut aus der Stichwunde nachgeflossen ist. Wenn hingegen nach der Wartezeit kein Blutkontakt detektiert wurde, kann die Messung durch die Kontrolleinrichtung 32 abgebrochen werden. Dadurch entstehen keine falschen Resultate durch fehlendes oder zu kleines Blutvolumen. Zweckmäßig gibt die Kontrolleinrichtung 32 eine entsprechende Meldung an den Benutzer aus.

Der Sammelvorgang braucht nicht statisch zu erfolgen, sondern kann im Zuge einer weiteren Rückbewegung des Stechelements 10 durchgeführt werden. Optional ist auch ein Sammeln auf der Hautoberfläche 34 möglich, wie es in Fig. 5 gezeigt ist. Hierbei kann ein Flüssigkeitskontakt durch das Stechorgan 16 als Messelektrode, welche in einen ausgetretenen Blutstropfen 50 eintaucht und damit in fluidischem Kontakt mit der Haut steht, und dem an die Haut anliegenden Andruckring 30 als Gegenelektrode zuverlässig erfasst werden. Der Andruckring ist zusätzlich dafür vorgesehen, den Blutfluss unter Druck zu unterstützen und die Wunde zu öffnen, so dass der Kontakt zwischen Stechorgan und Haut und damit der Schmerz für den Benutzer minimal ist.

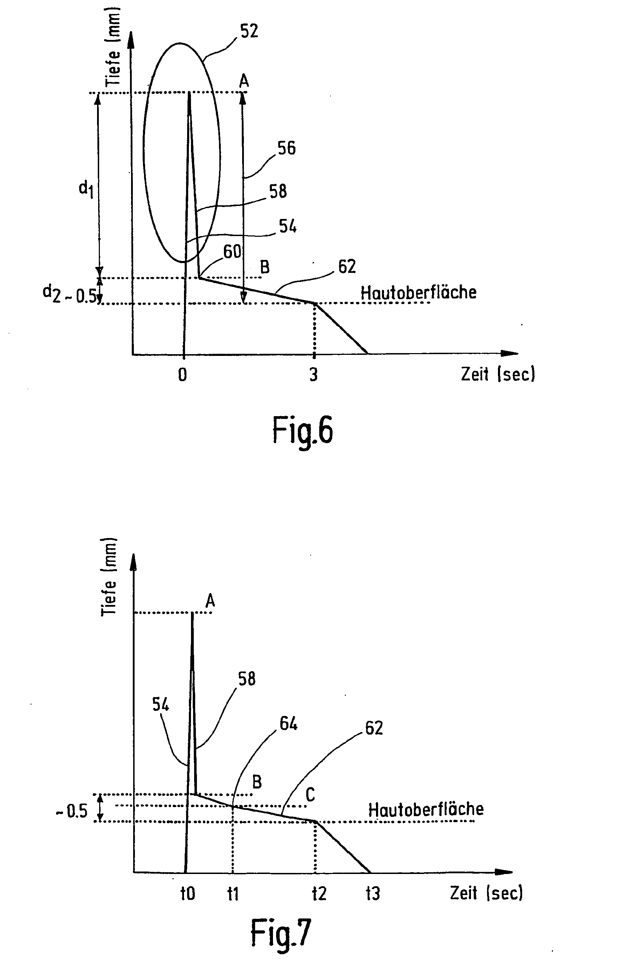

Für eine möglichst erfolgreiche und schmerzarme Blutaufnahme sind die in den Fig. 6 bis 9 gezeigten Stechprofile besonders vorteilhaft. Unter dem Begriff "Stechprofil" ist hierbei der Zeitverlauf der Stechbewegung zu verstehen, wie er als Funktion der Stechtiefe über der Zeit dargestellt ist.

Bei dem in Fig. 6 gezeigten Stechprofil wird das Stechorgan in den blutgebenden, schmerzempfindlichen Hautbereich 52 in einer Vorwärtsbewegung 54 bis zu einer vorgegebenen Tiefe 56 schnell eingestochen und sogleich in einer Rückziehbewegung 58 bis auf eine Tiefe von ca. 0,5 mm zurückgezogen. Die Rückziehphase liegt dabei in der Größenordnung der Dauer der Vorwärtsbewegung, d. h. wenigen 100 µs. Die Rückziehposition 60 kann sich im Bereich des Stratum Corneums und damit außerhalb des Schmerzbereichs 52 befinden. Anschließend folgt eine Sammelphase 62, in welcher Blut aufgenommen wird, während das Stechelement langsam bis auf die Hautoberfläche zurückgezogen wird. Diese Sammelphase 62 dauert einige Sekunden, wobei der durch das Stechorgan zurückgelegte Weg d2 wesentlich kleiner ist als der Rückziehweg d1 während der Rückziehphase 58. Durch das spätere langsamere Zurückziehen relaxiert die Haut, so dass der Stichkanal nicht sogleich wieder blockiert wird.

Da der Sammelvorgang eine gewisse Zeit benötigt, wird er zweckmäßigerweise in der nervenfreien äußeren Hautzone vollzogen. Das Sammeln unterhalb der Hautoberfläche, d.h. im eingestochenen Zustand, ist ebenfalls wichtig, um einen Blutaustritt auf die Haut zu vermeiden und somit einen besonders hygienischen Entnahmevorgang zu ermöglichen. Hinzu kommt, dass bereits auf die Hautoberfläche ausgetretenes Blut nur dann sauber mit dem Stechelement 10 entfernt werden kann, wenn die Kapillarkraft der Kapillare 18 an dieser Stelle genügend groß ist. Dies ist dann der Fall, wenn das Stechorgan 16 sich zumindest in leicht eingestochenem Zustand befindet, weil zu der Spitze hin die Kapillartiefe kleiner wird und damit die Kapillarkraft auch abnimmt.

Für das Stechprofil nach Fig. 6 sind elektrische oder elektromagnetische Antriebe besser geeignet als rein mechanische Antriebe, weil bei letzteren Prelleffekte schwerer zu kontrollieren sind (eine Dämpfung lässt sich einfacher elektrischer realisieren als durch eine mechanische Lösung).

Um die Stechtiefe definiert einstellen zu können, kann eine Detektion der Hautoberfläche beispielsweise durch eine Impedanzmessung vor dem eigentlichen Stechvorgang vorgenommen werden. Die Position der Hautoberfläche wird dabei während einer langsamen Vorwärtsbewegung des Stechelements 10 detektiert:

Das Umschalten zwischen den verschiedenen Bereichen des Stechprofils kann entweder durch die Position (Tiefe) gesteuert werden oder zeitgesteuert sein. Der Übergang zwischen den Bereichen mit unterschiedlichen Geschwindigkeiten kann durch eine unstetige Änderung erfolgen oder aber fließend sein (stetige Geschwindigkeitsänderung). Die stetige Lösung hat den Vorteil, dass weniger Antriebsenergie erforderlich ist und die Regelung der Bewegung vereinfacht wird.

Das in Fig. 7 gezeigte Stechprofil unterscheidet sich nur durch eine zusätzliche Stufe 64 während der Sammelphase 62. Hier erfolgt nach dem schnellen Zurückziehen bis Tiefe B ein etwas langsameres Zurückziehen bis Tiefe C, wobei sich während dieses Intervalls die Haut entspannt. Der eigentliche Sammelvorgang erfolgt dann nochmals verlangsamt von der Tiefe C bis an die Hautoberfläche. Dadurch kann eine Verkürzung des gesamten Stech-und Sammelvorgangs erreicht werden.

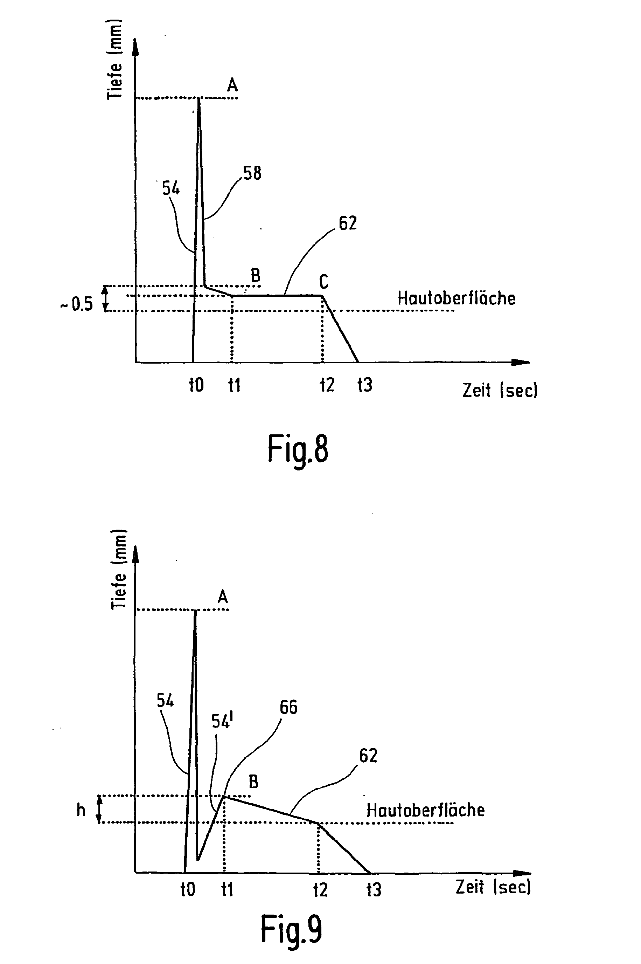

Eine ähnliche Ausführung sieht gemäß Fig. 8 vor, dass nach der schnellen Rückziehphase 58 die Hautentspannung durch langsames Zurückziehen des Stechorgans bis zum Zeitpunkt t1 erreicht wird, und anschließend bis zum Zeitpunkt t2 bei ruhendem Stechorgan weiter gesammelt wird. Hierbei ist allerdings eine seitliche Verschiebung des Stechorgans gegenüber der Haut für den Benutzer deutlicher zu spüren.

Bei dem Stechprofil nach Fig. 9 wird das Stechorgan nach einer ersten schnellen Vorwärtsbewegung 54 vollständig aus der Haut zurückgezogen und anschließend in einer zweiten Vorwärtsbewegung 54' mit geringerer Stechtiefe erneut eingestochen. In diesem Punkt wird dann ein Blutkontakt detektiert, und anschließend der Sammelvorgang 62 unter langsamem Zurückziehen des Stechorgans bis zur Hautoberfläche durchgeführt. Das vollständige Zurückziehen besitzt den Vorteil, dass ein eventuelles Nachprellen des Stechorgans außerhalb der Haut stattfindet und somit keine Dämpfung nötig ist, was die Anforderungen an die Aktuatorik stark reduziert.

Dieses Stechprofil bietet sich für eine einfache Regelungsvariante an. Zunächst kann vor der Zeit t0 die Hautoberfläche detektiert werden, um die Stechtiefe genau bestimmen zu können. Dieser Vorgang könnte induktiv oder durch eine Impedanzmessung erfolgen, indem wie oben beschrieben das Stechelement langsam gegen die Haut bewegt wird, bis deren Oberfläche angetastet wird. Der erste Stechvorgang (schnell hinein und schnell heraus) kann dann gleichsam "blind" erfolgen, d.h. ohne Rückkopplung durch einen Regelkreis. Dies hat den Vorteil, dass insbesondere im Falle der Verwendung eines elektromagnetischen Spulenantriebs eine schnelle und kostspielige Regelungselektronik vermieden werden kann. Bei einem Hybridantrieb würde dieser schnelle Stechvorgang vom mechanischen Teil übernommen (z. B. Feder- oder Kulissen-Antrieb). Nach dem ersten Stechvorgang wird das Stechorgan langsamer vorwärts bewegt als zuvor (Phase 54'). Diese Bewegung kann ebenfalls ohne Regelschleife erfolgen. Ist die Hautoberfläche erreicht (ggf. erfasst durch erneute Hautdetektion), so kann ein Steuersignal ausgelöst werden, welches den verbleibenden Abschnitt des Stechprofils (Sammelphase) triggert. Der Umkehrpunkt 66 kann gegenüber dem Triggersignal durch eine zeitliche Verzögerung bestimmt werden. Diese zeitliche Verzögerung muss kurz genug sein, um nicht wieder bis in die Schmerzzone 52 vorzudringen. Der Sammelvorgang bis t2 kann dann ebenfalls ohne Positionsregelung durch einfache Steuerung erfolgen. Anschließend wird das Stechelement bis zum Zeitpunkt t3 in das Gerät zurückgezogen und das Stechorgan somit von der Haut entfernt.

In Fig. 11 ist der zeitliche Verlauf des Messsignals 68 für verschiedene Möglichkeiten eines Blutkontakts in der Konfiguration nach Fig. 2 bis 4 näher veranschaulicht. Beim Einstich in trockene Haut nimmt die Leitfähigkeit bis zu einer Knickstelle 70 zunächst langsamer zu, bis ein Blutkontakt der Spitze 16 in der Dermis 36 zu einem raschen Signalanstieg führt. Am Ende der Vorwärtsbewegung wird ein Signalpegel 72 erreicht, der für einen direkten Flüssigkeitskontakt charakteristisch ist und somit als Vergleichssignal dienen kann. Hierbei ist zu berücksichtigen, dass beim Hautkontakt zunächst die Leitfähigkeit bzw. Impedanz der Hautfläche zwischen der Spitze 16 und der Ringelektrode 30 und somit der Ringradius maßgeblich ist. Beim Eintauchen der Spitze 16 in die Körperflüssigkeit 16 vergrößert sich die wirksame Elektrodenfläche schlagartig, und das Signal wird nur durch die im Vergleich zum Ringradius wesentlich geringere Dicke der Hautschicht zwischen der Blutzone 36 und dem darüber liegenden Ring 30 beeinflusst. Besonders für eine kapazitive Messung ist es daher vorteilhaft, wenn der Ring 30 gegenüber der Hautoberfläche 44 elektrisch isoliert ist.

Gemäß Fig. 11a wird somit am Ende der Vorwärtsbewegung, in der entsprechend Fig. 2 die blutgebende Zone 36 erreicht wird, der für einen Flüssigkeitskontakt charakteristische (und gegebenenfalls empirisch bestimmbare) Signalpegel 72 erreicht. Beim anschließenden Zurückziehen in die Sammelposition 40 gemäß Fig. 3 kann dann der Fall eintreten, dass eine kurze Zeit verstreicht, bis die Körperflüssigkeit bis zur zurückgezogenen Nadelspitze nachgeflossen ist. Dementsprechend nimmt das Signal beim Zurückziehen ab und erreicht beim zweiten Kontakt mit dem Blut wieder den Höchstwert 72. Somit kann nach einer vorgegebenen Wartezeit eine Erfolgskontrolle für die Blutaufnahme dahingehend durchgeführt werden, dass ein Vergleich durchgeführt wird, ob der hohe Signalpegel wieder vorliegt.

Für den Fall eines schnellen Nachfließens der Körperflüssigkeit reißt der Flüssigkeitskontakt zu der zurückbewegten Spitze 16 nicht ab, und der Signalpegel bleibt konstant hoch, wie es in Fig. 11b gezeigt ist. Auch hier ist eine positive Erfolgskontrolle möglich.

Hingegen nimmt entsprechend Fig. 11c bei nicht oder nur schlecht nachfließendem Blut der Signalpegel auch in der zurückgezogenen Position weiter ab, so dass aufgrund der Änderung des Messsignals nach einer gegebenen Wartezeit das Vorliegen eines Fehlerfalls erkannt wird, in welchem ein Sammeln der Körperflüssigkeit vorrausichtlich nicht erfolgreich ist. Eine kritische Signaländerung kann durch einen Vergleich mit dem Signalpegel am Anfang der Wartezeit oder durch eine Auswertung der Steigung des Signalverlaufs bestimmt werden. Demgemäß kann die Messung abgebrochen und der Benutzer gewarnt werden.

## Patentansprüche

1. Vorrichtung zur Entnahme von Körperflüssigkeit, insbesondere Blut, mit einem eine Kapillarstruktur (18) zur Aufnahme der Körperflüssigkeit aufweisenden Stechelement (10) zum Einstechen durch die Haut in ein Körperteil (14) und einem Stechantrieb (22) für eine Vorwärts- und Rückbewegung des Stechelements (10), **dadurch gekennzeichnet, dass** der Stechantrieb (22) zur Rückbewegung des Stechelements (10) in einer Rückziehphase (58) um einen ersten Weg (d1) und in einer späteren Sammelphase (62) um einen zweiten Weg (d2) ausgebildet ist, wobei die Rückziehphase (58) kürzer als die Sammelphase (62) und der erste Weg (d1) größer als der zweite Weg (d2) und der zweite Weg großer als Null ist.

2. Vorrichtung nach Anspruch 1, **dadurch gekennzeichnet, dass** die Rückziehphase (58) in der Größenordnung der Dauer der Vorwärtsbewegung (54) liegt.

3. Vorrichtung nach Anspruch 1 oder 2, **dadurch gekennzeichnet, dass** die Sammelphase (62) gegenüber der Rückziehphase (58) um das 10-bis 10.000-fache verlängert ist.

4. Vorrichtung nach einem der Ansprüche 1 bis 3, **dadurch gekennzeichnet, dass** der Stechantrieb (22) das Stechelement (10) während der Sammelphase (62) vorzugsweise mit gleichförmiger Geschwindigkeit zurückzleht.

5. Vorrichtung nach einem der Ansprüche 1 bis 4, **dadurch gekennzeichnet, dass** der Stechantrieb (22) das Stechelement (10) in einem ersten Zeitabschnitt der Sammelphase (62) gegenüber einem nachfolgenden zweiten Zeitabschnitt schneller zurückzieht.

6. Vorrichtung nach einem der Ansprüche 1 bis 5, **dadurch gekennzeichnet, dass** der Stechantrieb (22) das Stechelement (10) in dem zweiten Zeitabschnitt für eine bestimmte Verweilzeit anhält.

7. Vorrichtung nach einem der Ansprüche 1 bis 6, **dadurch gekennzeichnet, dass** der Stechantrieb (22) das Stechelement (10) während der Rückziehphase (58) vollständig aus der Haut zurückzieht und vor der Sammelphase (62) mit geringerer Stechtiefe erneut einsticht.

8. Vorrichtung nach einem der Ansprüche 1 bis 7, **dadurch gekennzeichnet, dass** der Stechantrieb (22) das Stechelement (10) während der Sammelphase (62) um einen zweiten Weg zwischen 2 mm und 0,1 mm zurückbewegt.

9. Vorrichtung nach einem der Ansprüche 1 bis 8, **dadurch gekennzeichnet, dass** die Bewegung des Stechelements (10) durch eine Zeit-oder Positionserfassung gesteuert ist.

10. Vorrichtung nach einem der Ansprüche 1 bis 9, **gekennzeichnet durch** ein Detektionsmittel (24), welches einen Körperflüssigkeftskontakt des Stechelements (10) zumindest am Beginn und Ende einer Wartezeit nach der Vorwärtsbewegung (54) während einer Sammelphase (62) für eine Erfolgskontrolle **durch** ein Messsignal erfasst.

11. Vorrichtung nach Anspruch 10. **dadurch gekennzeichnet, dass** das Stechelement (10) mittels des Stechantriebs (22) nach der Vorwärtsbewegung (54) in einer Rückziehbewegung aus der tiefsten Einstechposition in eine Rückziehposition von geringerer Stechtiefe zurückziehbar ist, und dass das Detektionsmittel (24) in der Rückziehposition bei einem Körpertlüssigkeitskontakt anspricht.

12. Vorrichtung nach Anspruch 10 oder 11, **dadurch gekennzeichnet, dass** der Körpertlüssigkeitskontaki nach der Vorwärtsbewegung (54) vorzugsweise innerhalb des Körperteils (14) erfasst wird, wobei eine gegebene Signalveränderung als Fehlerfall erkannt wird.

13. Vorrichtung nach einem der Ansprüche 10 bis 12, **dadurch gekennzeichnet, dass** der Stechantrieb (22) dazu ausgebildet ist, das Stechelement (10) nach einer ersten Vorwärtsbewegung (54) aus der Haut zurückzuziehen und sogleich in einer zweiten Vorwärtsbewegung (54) mit geringerer Stechtiefe erneut einzustechen, und dass anschließend das Detektionsmittel (24) bei einem Körperflüssigkeitskontakt anspricht.

14. Vorrichtung nach einem der Ansprüche 1 bis 13, **dadurch gekennzeichnet, dass** der Stechantrieb (22) zweistufig ausgebildet ist, wobei eine vorzugsweise mechanisch arbeitende erste Antriebsstufe für eine schnelle Bewegung und eine vorzugsweise elektrisch arbeitende zweite Antriebsstufe für eine langsamere geregelte Bewegung des Stechelements (10) vorgesehen ist.

15. Vorrichtung nach einem der Ansprüche 10 bis 14, **dadurch gekennzeichnet, dass** der Körperflüssigkeitskontakt über das Stechelement (10) und ein in Anlage mit dem Körperteil (14) stehendes, vorzugsweise elektrisch isoliertes Kontaktelement (30) detektierbar ist.

16. Vorrichtung nach einem der Ansprüche 10 bis 15, **gekennzeichnet durch** Elektroden (10,30) als Detektionsmittel zur Erfassung eines Körperflüssigkeitskontakts des Stechelements (10) auf der Haut außerhalb des Körperteils (14), wobei die Elektroden **durch** das über die Körperflüssigkeit in fluidischen Kontakt mit der Haut befindliche Stechelement (10) und ein in Anlage mit der Haut stehendes Kontaktelement (30) gebildet sind.

17. Vorrichtung nach einem der Ansprüche 10. bis 16, **dadurch gekennzeichnet, dass** die Detektionsmittel (10,30;24) den Körperflüssigkehskontakt bei Erreichen eines gegebenenfalls empirisch ermittelten Signalpegels eines elektrischen Messparameters insbesondere kapazitiv oder induktiv oder resistiv erfassen.

18. Vorrichtung nach einem der Ansprüche 10 bis 17, **dadurch gekennzeichnet, dass** der Körperflüssigkeitskontakt über das Stechelement (10) durch eine Impedanzmessung gegenüber der Haut erfassbar ist.

19. Vorrichtung nach einem der Ansprüche 10 bis 18, **dadurch gekennzeichnet, dass** der Körperflüssigkeitskontakt über optische Detektionsmittel vorzugsweise durch die Kapillarstruktur (18) oder einen Leichtleiter hindurch innerhalb des Körperteils (14) erfassbar ist.

20. Vorrichtung nach einem der Ansprüche 10 bis 19, **gekennzeichnet durch** eine Kontrolleinrichtung (32) zur Erfolgskontrolle der Flüssigkeitsaufnahme nach Maßgabe des detektierten Körperflüssigkeftskontakts.

21. Vorrichtung nach Anspruch 20, **dadurch gekennzeichnet, dass** die Kontrolleinrichtung (32) bei ausbleibendem Körperflüssigkeitskontakt ein Fehlersignal generiert und den Entnahmevorgang abbricht.

## Claims

1. Device for withdrawing body fluid and in particular blood comprising a lancing element (10) having a capillary structure (18) to collect the body fluid and for insertion through the skin into a body part (14) and a lancing drive (22) for a forward and backward movement of the lancing element (10) **characterized in that** the lancing drive (22) is designed to move the lancing element (10) back by a first distance (d1) in a retraction phase (58) and by a second distance (d2) in a later collection phase (62), wherein the retraction phase (58) is shorter than the collection phase (62) and the first distance (d1) is larger than the second distance (d2) and the second distance is larger than zero.

2. Device according to claim 1, **characterized in that** the duration of the retraction phase (58) is of the same order of magnitude as the duration of the forward movement (54).

3. Device according to claim 1 or 2, **characterized in that** the collection phase (62) is 10- to 10,000-fold longer than the retraction phase (58).

4. Device according to one of the claims 1 to 3, **characterized in that** the lancing drive (22) retracts the lancing element (10) during the collection phase (62) preferably at a uniform speed.

5. Device according to one of the claims 1 to 4, **characterized in that** the lancing drive (22) retracts the lancing element (10) more rapidly in a first time segment of the collection phase (62) compared to a subsequent second time segment.

6. Device according to one of the claims 1 to 5, **characterized in that** the lancing drive (22) stops the lancing element (10) in the second time segment for a certain holding time.

7. Device according to one of the claims 1 to 6, **characterized in that** the lancing drive (22) completely pulls the lancing element (10) out of the skin during the retraction phase (58) and inserts it again to a lesser lancing depth before the collection phase (62).

8. Device according to one of the claims 1 to 7, **characterized in that** the lancing element (10) is moved back by the lancing drive (22) by a second distance of between 2 mm and 0.1 mm during the collection phase (62).

9. Device according to one of the claims 1 to 8, **characterized in that** the movement of the lancing element (10) is controlled by a registration of time or position.

10. Device according to one of claims 1 to 9, **characterized in that** a detection means (24) which detects contact of the lancing element (10) with body fluid by a measurement signal during a collection phase (62) at least at the beginning and at the end of a waiting period after the forward movement (54) in order to check that it has been successful.

11. Device according to claim 10, **characterized in that** after the forward movement (54), the lancing element (10) can be pulled back in a retraction movement out of the deepest lancing position into a retraction position of lesser lancing depth by means of the lancing drive (22), and that the detection means (24) responds when there is a contact with body fluid in the retraction position.

12. Device according to claim 10 or 11, **characterized in that** contact with body fluid is detected after the forward movement (54) preferably within the body part (14) wherein a given change in signal is identified as a fault.

13. Device according to one of the claims 10 to 12, **characterized in that** the lancing drive (22) is designed to retract the lancing element (10) from the skin after a first forward movement (54) and to immediately insert it again in a second forward movement (54) of lesser lancing depth, and that subsequently the detection means (24) responds when there is contact with body fluid.

14. Device according to one of the claims 1 to 13, **characterized in that** the lancing drive (22) is designed in two stages wherein a first drive stage which preferably operates mechanically is provided for a rapid movement and a second drive stage which preferably operates electrically is provided for a slower controlled movement of the lancing element (10).

15. Device according to one of the claims 10 to 14, **characterized in that** contact with body fluid can be detected by the lancing element (10) and a preferably electrically insulated contact element (30) which is in contact with the body part (14).

16. Device according to one of the claims 10 to 15, **characterized by** electrodes (10, 30) as detection means for detecting a contact of the lancing element (10) with body fluid on the skin outside of the body part (14) wherein the electrodes are formed by the lancing element (10) which is in fluidic contact with the skin via the body fluid and a contact element (30) which is in contact with the skin.

17. Device according to one of the claims 10 to 16, **characterized in that** the detection means (10, 30, 24) detect the contact with body fluid when a signal level that is optionally determined empirically of an electrical measurement parameter and in particular capacitance or inductance or resistance is reached.

18. Device according to one of the claims 10 to 17, **characterized in that** the contact with body fluid can be detected by the lancing element (10) by an impedance measurement relative to the skin.

19. Device according to one of the claims 10 to 18, **characterized in that** the contact with body fluid can be detected by optical detection means within the body part (14) preferably through the capillary structure (18) or through a light guide.

20. Device according to one of the claims 10 to 19, **characterized by** a control device (32) for checking the success of liquid collection according to the detected contact with body fluid.

21. Device according to claim 20, **characterized in that** the control device (32) generates an error signal and terminates the withdrawal process in the absence of a contact with body fluid.

## Revendications

1. Dispositif pour prélever un liquide corporel, notamment du sang, comprenant un élément de piqûre (10) comprenant une structure capillaire (18) pour recevoir le liquide corporel, servant à piquer à travers la peau dans une partie du corps (14), et une commande de piqûre (22) pour un déplacement vers l'avant et vers l'arrière de l'élément de piqûre (10), **caractérisé en ce que** la commande de piqûre (22) est exécutée pour le déplacement vers l'arrière de l'élément de piqûre (10) sur une première distance (d1) dans une phase de retrait (58) et sur une deuxième distance (d2) dans une phase de recueil (62) ultérieure, la phase de retrait (58) étant plus courte que la phase de recueil (62) et la première distance (d1) étant supérieure à la deuxième distance (d2) et la deuxième distance étant supérieure à zéro.

2. Dispositif selon la revendication 1, **caractérisé en ce que** la phase de retrait (58) a une durée de l'ordre de grandeur du déplacement vers l'avant (54).

3. Dispositif selon la revendication 1 ou 2, **caractérisé en ce que** la phase de prélèvement (62) est de 10 à 10 000 fois plus longue que la phase de retrait (58).

4. Dispositif selon l'une des revendications 1 à 3, **caractérisé en ce que** la commande de piqûre (22) retire l'élément de piqûre (10) avec une vitesse de préférence uniforme pendant la phase de recueil (62).

5. Dispositif selon l'une des revendications 1 à 4, **caractérisé en ce que** la commande de piqûre (22) retire l'élément de piqûre (10) plus rapidement dans un premier intervalle de temps de la phase de recueil (62) que dans un second intervalle de temps consécutif.

6. Dispositif selon l'une des revendications 1 à 5, **caractérisé en ce que** la commande de piqûre (22) retient l'élément de piqûre (10) pendant un temps d'attente déterminé dans le second intervalle de temps.

7. Dispositif selon l'une des revendications 1 à 6, **caractérisé en ce que** la commande de piqûre (22) retire complètement l'élément de piqûre (10) de la peau pendant la phase de retrait (58) et le réintroduit avec une profondeur de piqûre plus réduite avant la phase de recueil (62).

8. Dispositif selon l'une des revendications 1 à 7, **caractérisé en ce que** la commande de piqûre (22) ramène l'élément de piqûre (10) sur une deuxième distance comprise entre 2 mm et 0,1 mm pendant la phase de recueil (62).

9. Dispositif selon l'une des revendications 1 à 8, **caractérisé en ce que** le déplacement de l'élément de piqûre (10) est commandé par un dispositif de saisie de temps ou de position.

10. Dispositif selon l'une des revendications 1 à 9, **caractérisé par** un moyen de détection (24) qui détecte, grâce à un signal de mesure, un contact de l'élément de piqûre (10) avec un liquide corporel au moins au début à la fin d'un temps d'attente après le déplacement vers l'avant (54) pendant une phase de recueil (62) pour un contrôle d'efficacité.

11. Dispositif selon la revendication 10, **caractérisé en ce que** l'élément de piqûre (10) peut, après le déplacement vers l'avant (54), être retiré au moyen de la commande de piqûre (22) de la position de piqûre la plus profonde dans une position de retrait d'une profondeur de piqûre plus réduite au cours d'un mouvement de retrait et **en ce que** le moyen de détection (24) répond, dans la position de retrait, en cas contact avec un liquide corporel.

12. Dispositif selon la revendication 10 ou 11, **caractérisé en ce que** le contact avec un liquide corporel est détecté de préférence à l'intérieur de la partie du corps (14) après le déplacement vers l'avant (54), une modification de signal donnée étant reconnue comme une défaillance.

13. Dispositif selon l'une des revendications 10 à 12, **caractérisé en ce que** la commande de piqûre (22) est exécutée pour retirer l'élément de piqûre (10) de la peau après un premier déplacement vers l'avant (54) et repiquer aussitôt avec une profondeur de piqûre plus réduite au cours d'un deuxième déplacement vers l'avant (54) et **en ce que** le moyen de détection (24) répond ensuite en cas de contact avec un liquide corporel.

14. Dispositif selon l'une des revendications 1 à 13, **caractérisé en ce que** la commande de piqûre (22) est exécutée de manière à être à deux niveaux, un premier niveau de commande à fonctionnement de préférence mécanique étant prévu pour un mouvement rapide et un second niveau de commande à fonctionnement de préférence électrique étant prévu pour un mouvement réglé plus lent de l'élément de piqûre (10).

15. Dispositif selon l'une des revendications 10 à 14, **caractérisé en ce que** le contact avec un liquide corporel peut être détecté par le biais de l'élément de piqûre (10) et d'un élément de contact (30), de préférence électriquement isolé, placé au contact de la partie du corps (14).

16. Dispositif selon l'une des revendications 10 à 15, **caractérisé par** des électrodes (10, 30) en tant que moyen de détection pour détecter un contact de l'élément de piqûre (10) avec un liquide corporel sur la peau à l'extérieur de la partie du corps (14), les électrodes étant formées par l'élément de piqûre (10) qui se trouve en contact fluide avec la peau par le biais du liquide corporel et un élément de contact (30) placé au contact de la peau.

17. Dispositif selon l'une des revendications 10 à 16, **caractérisé en ce que** les moyens de détection (10, 30; 24) détectent le contact avec un liquide corporel de manière notamment capacitive ou inductive ou résistive lorsqu'est atteint un seuil de signal d'un paramètre de mesure électrique déterminé le cas échéant de manière empirique.

18. Dispositif selon l'une des revendications 10 à 17, **caractérisé en ce que** le contact avec un liquide corporel peut être détecté par le biais de l'élément de piqûre (10) grâce à une mesure d'impédance par rapport à la peau.

19. Dispositif selon l'une des revendications 10 à 18, **caractérisé en ce que** le contact avec un liquide corporel peut être détecté à l'intérieur de la partie du corps (14), de préférence à travers la structure capillaire (18) ou un conducteur de lumière, par le biais de moyens de détection optique.

20. Dispositif selon l'une des revendications 10 à 19, **caractérisé par** un dispositif de contrôle (32) pour le contrôle d'efficacité du prélèvement de liquide en fonction du contact avec un liquide corporel détecté.

21. Dispositif selon la revendication 20, **caractérisé en ce que** le dispositif de contrôle (32) génère un signal d'erreur en cas d'absence de contact avec un liquide corporel et interrompt le processus de prélèvement.
